# EUROPEAN PATENT APPLICATION

(11) **EP 4 582 119 A2**
(43) Date of publication of application: **09.07.2025**
(21) Application number: 25178009.4
(22) Date of filing: 29.04.2022
(51) Int. Cl.: A61M 5/145

(54) **NEUROSURGICAL DEVICE**

(30) Priority: 30.04.2021 GB 202106203; 30.04.2021 GB 202106210; 30.04.2021 GB 202106224
(62) Divisional of application: 22721111.7
(71) Applicant: Neurochase Technologies Limited, Bristol BS1 5QT (GB)
(72) Inventor: GILL, Steven Streatfield, Bristol, BS8 3HP (GB); GILL, Thomas, Bristol, BS8 3HP (GB)
(74) Representative: J A Kemp LLP

(57) **Abstract**

A cannula for insertion through a guide tube into the brain, to deliver an infusate to a target brain volume, wherein the cannula comprises a bubble vent configured to prevent gas and/or micro-organisms from entering the cannula. Also provided is a kit for convection enhanced delivery of an infusate to the brain parenchyma.

## Description

### FIELD

The present invention relates to apparatus for use in neurosurgery. Particularly, the invention relates to an apparatus used to deliver therapeutic agents by infusion directly into the brain parenchyma.

### BACKGROUND

Treatment of neurological diseases can be hindered by the presence of the blood-brain barrier. It can be difficult to develop therapeutic agents that can be delivered from the systemic circulation into the brain parenchyma. It can be desirable to deliver therapeutic agents to specific regions of the brain ('brain volumes' or 'target volumes'). Obtaining an appropriate concentration of the therapeutic agent in a target volume whilst minimising exposure of the rest of the brain to the therapeutic agent is desirable, to reduce undesired side effects.

Convection Enhanced Delivery (CED) is a method of targeted delivery of therapeutic agents to particular brain volumes by the controlled infusion of the therapeutic agent delivered into the brain parenchyma in a fluid using extremely small cannulas or tubing (often referred to in the art as micro-catheters). The micro-catheters have a port or ports at their distal end, allowing an infusate including the therapeutic agent to exit the catheter into the target brain volume. A continuous pressure gradient must be achieved at the port to overcome the ambient pressure of the target brain volume, thereby allowing the infusate to flow effectively into the target brain volume.

There are challenges in using the CED technique because fluid flowing from a cannula port will follow the path of least resistance. Typically the path of least resistance will be back along the cannula/ tissue interface causing so-called reflux, instead of driving the fluid into the tissue as desired. In order to control reflux, so that fluid is driven into the tissue of the target volume, CED cannulas typically have an abrupt change in diameter (a 'step') towards the distal end. For example the distal end of the cannula may be of less than 1mm in diameter for a short length to the extreme end, with a step up to a diameter of 1.5 to 2.5 mm for the remainder of the cannula. The small diameter distal end, if inserted atraumatically creates a tissue seal around the cannula, minimising reflux along the interface. The change in diameter at the step compresses tissue on insertion into the brain and further resists reflux.

Careful control of flow rates, typically in the range of 3-5 microlitres per minute, facilitates flow of an infusate into the tissues in preference to reflux. Higher flow rates will tend to increase the degree of reflux and may lead to refluxing infusate getting past the step and flowing back along the cannula/brain tissue interface, even at the wider diameter portion of the cannula. When the step is overwhelmed in this way, the infusate enters a low resistance pathway through the larger circumferential space around the larger diameter part of the cannula, with a resultant reduction of the intended distribution of infusate within the target volume.

The design of the reflux resistant step differs between CED cannulas. The cannula described in EP 1482851B1 and US patent application 2010/0217228 A1 provide a single step, WO2007024841A2 provides a cannula with a plurality of steps and WO2014-016591 has a recessed step.

In addition to the step design the length that a cannula extends beyond the step, to its extreme distal end, is an important determinant of both the volume and shape of distribution of infusate into brain tissue. A short-stepped cannula (i.e. a cannula with only a short length of smaller diameter) will have a small volume of distribution (Vd) that is roughly spherical. As the length of the smaller diameter portion increases the Vd becomes larger and more ovoid, then cylindrical and then pear shaped with a bulbous distal end.

When the radius of distribution of fluid convected into the brain exceeds that of the region of compressed tissue at the step then the infusate will tend to enter the low resistance pathway along the larger diameter portion of the cannula and reflux away from the target volume. This places a limit on the Vd at the target.

To deliver therapy to clinically meaningful tissue volumes with a fixed step length arrangement can therefore be problematic. Using a cannula with a short fixed step-length will require sequential infusions made at different points along a trajectory into the brain in order to fill an elongate volume of tissue. Similarly multiple passes are required to fill a larger more spherical structure in the brain. Conversely filling a smaller target using a catheter with a relatively long 'step length' may make it difficult to contain the therapy in the target. Therefore the ability to adjust the step length for each target provides significant advantages.

The majority of targets for treating CNS (central nervous system) diseases by CED require several cannulas to be implanted to achieve the desired coverage of infusate into brain tissue. When the desired target volume and shape has been defined from MRI images, the number and orientation of cannulas required to fill the volume are determined by understanding the likely distribution shape and volume that can be achieved with the cannulas being deployed. It is also important to confirm that the infused therapy has covered the prescribed treatment volume with MRI imaging either during or immediately following the infusion. When rigid cannulas are used they are fixed in a stereotactic frame whilst the infusion is carried out. In such arrangements the stereotactic frame needs to be MRI compatible and have a low profile to fit in the imaging coil. Typically, additional cannula placements and infusions are required after completion of the first. Thus such procedures are relatively lengthy and can expose the patient to increased risks from prolonged anaesthesia and immobilisation.

Alternative procedures employing multiple cannula trajectories make use of flexible cannulas that are implanted and secured to the skull. The flexible cannula tubing extending out of the skull can be attached to a low profile skull fixation. This facilitates safe transfer into an MRI scanner where simultaneous infusions can be conducted with the patient awake for neurological evaluation. A stereotactic frame is not required during infusion where flexible cannulas are employed. The flexible cannulas may be removed after the infusions are complete, or in some instances may be left in-situ for repeated infusions days, weeks or months later.

Uses of flexible cannulas are described in EP 1482851B1, EP13001067.1, EP2819739B1, and WO2014-016591. In these arrangements, the distance between the cannula's distal end and the reflux resistant step can be adjusted. In each case the flexible cannula has a proximal hub and is cut to a desired length for insertion to a target point in the brain. When inserted into an implanted guide tube, also cut to a desired length, the cannula's hub acts as a stop when it engages with a proximal head on the guide tube that is fixed in the skull. The cannula extends beyond the guide tube and the step thus created by the change in diameter from the cannula to the guide tube provides resistance to reflux of infusate.

In typical procedures to implant flexible cannulas described in EP 1482851B1, EP13001067.1 and EP2819739B1, a profiled hole is made in the skull along the selected trajectory, guided by a stereo guide or image guided robot. A probe is then passed through the hole to the planned distal end of the guide tube and then withdrawn, leaving a track in brain tissue. The guide tube, cut to length, is placed over a delivery probe such that the rounded tip of the probe extends just beyond its distal end. The guide tube is inserted down the pre-made track until the head on its proximal end press fits into the formed hole in the skull. The probe is now advanced to the planned position of the cannula target and then withdrawn leaving a track through the tissue that is contiguous with the bore of the guide tube.

The cannula, connected to an infusion pump and delivering infusate at a low flow rate is inserted down the guide tube and through the pre-formed track in the tissue. The slow infusion through the cannula prevents coring of tissue during its transit. When a therapeutic fluid is delivered through the cannula's distal port it will follow the path of least resistance and flow back along the cannula-tissue interface before meeting the region of tissue compressed by the distal end of the guide tube. The localised pressure on the interface acts to inhibit reflux and the infusate is then preferentially driven radially into the tissue.

The devices described above have potential disadvantages. The method of insertion can cause micro trauma to the tissues, creating a low resistance path for infusate which impairs reflux control at the guide tube- tissue interface. As the guide tube is inserted into the pre-made track in the brain the cut edges of its distal end may tend to shear the tissue creating a circumferential column of fragmented tissue. The force required to push fit the head of the guide tube into the pre formed hole in the skull is also transferred to the distal end of the guide tube, which may add to the localised tissue trauma in the region of the step.

Additionally, as the cannula is inserted down the guide tube it will tend to act as a piston and drive a column of air ahead of it. Even with the application of suction at the proximal end of the guide tube it is difficult to vent air through the narrow space between the cannula and the guide tube. If air is driven into the brain, it tears the tissue and creates a space occupying lesion in close proximity to the reflux controlling step.

In the short term this is likely to disrupt the intended pattern of distribution of the infusate, but as the air is absorbed the cavity left can provide a low resistance pathway at the step, which will augment undesired reflux.

EP3119310B1 describes a guide tube with an internal profile configured to provide a fluid return path for carrying any fluid displaced from within the guide tube during insertion of a catheter. Such a path may also conduct and vent air during catheter insertion. However, to create a profile with internal channels of sufficient size to facilitate air venting increases the overall dimensions of the guide tube. A wider diameter guide tube may cause more trauma on insertion. Narrow channels between the guide tube and cannula may be liable to obstruction of air flow by the presence of liquid, due to surface tension.

International Patent Application WO2014/016591A1 discloses a recessed step arrangement, wherein the guide tube comprises an internal recess that compresses tissue therein. The internal recess provides a step feature that is intended to provide more effective compression of tissue to limit the flow of infusate along the cannula-tissue interface. However, in such an arrangement the guide tube cores a portion of brain tissue on insertion. The local tissue trauma could result in neurological deficits if in an eloquent part of the brain or may cause haemorrhage.

A further potential difficulty with known CED devices, particularly those that are chronically implanted is brain movement. The brain moves within the skull so that fixing of a cannula and/or associated guide tubes to the skull can cause movement of the tubing or cannula relative to brain tissue. This may cause local tissue trauma, particularly at a reflux resistant step, creating local vacuolation and the creation of a low resistance pathway that will tend to augment rather than resist reflux.

It is an aim of the invention to address at least some of the aforementioned difficulties, and in particular to provide a CED apparatus with improved reflux resistance and reduced trauma in use.

### SUMMARY

According to a first aspect, the present invention provides a neurosurgical apparatus for convection enhanced delivery of an infusate to the brain parenchyma, the neurosurgical apparatus comprising: a guide tube for insertion into the brain and having a proximal end, a distal end and a through-bore for passage of a cannula; wherein at least an outer layer of the guide tube is of a hydrophobic material that is resiliently deformable and porous, to allow passage of air.

According to a second aspect the present invention also provides a guide tube for use in the neurosurgical apparatus according to the first aspect. The guide tube is for insertion into the brain and has a proximal end, a distal end and a through-bore for passage of a cannula; wherein at least an outer layer the guide tube is of a hydrophobic material that is resiliently deformable and porous to allow passage of air.

By hydrophobic is meant that the static contact angle θ, at the liquid vapour interface of a water droplet on the surface of the material employed to form the guide tube, is greater than 90°. Advantageously at least the outer layer of the material of the guide tube disclosed herein is superhydrophobic, having θ > 150°.

The use of the neurosurgical apparatus and in particular the guide tube of the invention will be described in more detail hereafter with reference to methods of convection enhanced delivery of an infusate to the brain. However, the guide tube can find use in any surgical procedure where delivery of a small instrument or tool to the brain (such as a cannula, especially a 'micro-catheter') is envisaged.

The porous and hydrophobic or superhydrophobic outer layer of the guide tube provides a venting route for air as described in more detail hereafter. The hydrophobic or superhydrophobic property enables the separation of air bubbles from the cerebrospinal fluid (CSF), and from the extracellular fluid of a patient, or from an aqueous infusate fluid, at the interface with the material of the outer layer. Aqueous fluid is effectively repelled from the hydrophobic outer layer preventing wetting of the material of the guide tube, maintaining porosity to air as well as increasing the effectiveness of the guide tube in repelling fluid from the guide-tube / tissue interface and limiting reflux. The use of a porous material to vent air in this way also removes the need to provide special channels or similar structures within the walls of the guide tube to vent air, as are used in some of the prior art devices discussed above. This allows the cross-sectional area of the guide tube to be reduced significantly, by more than 50% compared to some prior art devices. This greatly reduces trauma to brain tissue and the likelihood of unwanted complications.

At least the outer layer of the guide tube is resiliently deformable. In use the guide tube, fitted about a guide tube probe passing through its throughbore, can be inserted into a pre-made track made within the brain. The pre-made track is of a narrower diameter than the guide tube. The resiliently deformable material provides advantages. The extreme distal end of the guide tube, typically a square cut end, will tend to deform to, or towards, a "bullet shape" that passes more easily down the pre-made track than prior art devices - where a square cut tube end will tend to cause trauma by cutting into brain tissue.

When no longer being inserted the bullet shaped extreme distal end will tend to return to or towards its original (e.g. square cut) form. This extreme end can act as a physical barrier to reflux in the known manner. However, the effectiveness against reflux is enhanced by the use of hydrophobic material and by the elasticity provided by the resiliently deformable material employed.

When inserted in brain tissue the guide tube, along its length, is subject to inwards radial compression from brain tissue as it is passed down the track. Thus, when in position, the guide tube will tend to expand radially outwards, back towards its original diameter, thereby pressing against the brain tissue. This can provide a good sealing interface with the brain tissue. The guide tube acts to prevent reflux along its length within the brain, as described in more detail hereafter.

To prevent unwanted trauma to brain tissue, the compression applied to the brain tissue by the implanted guide tube should avoid being significantly greater than the elastic limit generally expected for brain tissue. This can be achieved by suitable choice of materials and diameter of pre-made track and guide tube. The Youngs modulus of suitable materials may be in the range of say 10kPa to 1MPa.

Suitable materials of construction for at least the outer layer of the guide tube include, ePTFE, silicone foams, polyurethane foams, shape memory polymers, polymers extruded as microporous hollow fibres or electrospun polymers. Electrospun polymers and polymers extruded as microporous hollow fibres may include but are not limited to PTFE (polytetrafluoroethylene), PVDF (polyvinylidene difluoride), PU (polyurethane), polypropylene or mixtures and/or copolymers thereof. Shape memory polymers may be employed. Where foams are employed at least a portion of the cells of the foam are open cell to allow passage of air through the bulk material. All or substantially all of a foam construction outer layer or guide tube may be an open cell foam.

An additional advantage of the use of a porous material for at least the outer layer of the guide tube is that it may provide the opportunity of integration with brain tissue over time. i.e. the brain tissue may enter the porous structure of the guide tube. This can aid in securing the guide tube in its desired location, even if the compressive force between brain tissue and guide tube reduces over time. Integration of the guide tube and brain tissue can remove the sharply defined cannula-tissue interface and thereby will reduce the likelihood of reflux.

At least the outer layer of the guide tube may be of a material that has been post processed to improve its integration capability or other desirable properties. For example, the outer layer may comprise a coating and/or surface treatment configured to improve lubricity and/or to promote tissue integration. The surface treatment may comprise a plasma surface treatment. The coating may improve its lubricity or enhance its hydrophobicity. The coating may comprise a hydrophilic material.

The guide tube may further comprise an outermost layer axially outward of the outer layer, wherein the outermost layer comprises a hydrophilic material. The outermost layer of hydrophilic material may contribute to improving the lubricity of the guide tube. The outermost layer may comprise a mixture of hydrophilic and hydrophobic materials. Different cell types in the brain integrate more easily with hydrophobic or hydrophilic materials. For example, neurons bond more easily to hydrophobic materials, while glial cells bond more easily to hydrophilic materials. An outermost layer comprising a mixture of these materials may improve tissue integration with multiple cell types.

At least the outer layer of the guide tube is resiliently deformable to minimise trauma on insertion into brain tissue. However, if the guide tube is insufficiently stiff, it may be distorted, in particular shortened in length, as it is inserted into brain tissue, even with the aid of a guide tube probe in the through bore. However, brain tissue is a relatively compliant (easily deformed) material. A guide tube made only of material that approaches the compliance of brain tissue may be difficult to insert successfully.

Therefore, the guide tube may be constructed of a non-homogeneous material and/or of a plurality of materials with different properties. This can provide a desired combination of stiffness in the axial direction and compliance (resiliently deformable behaviour) in the radial direction.

Where a guide tube is constructed of a non-homogeneous material and/or a plurality of materials with different properties (in particular stiffness), the degree of stiffening provided may be along the entire or substantially the entire length of the guide tube. However, in some embodiments of the invention, the provision of stiffening may be varied along the length of the guide tube. For example, and as discussed further below, there may be reduced stiffening or even no additional stiffening provided at the proximal end of the guide tube.

For example, a guide tube may be constructed of a foam having increasing density from the outside radially inwards towards the throughbore, or a region of increased density at or near the throughbore. The increased density provides increased stiffness, thereby providing support to a more compliant outer region.

Alternatively or additionally, the guide tube may have a laminated structure with a stiffer layer or layers at or near the throughbore. This provides support to a more compliant outer layer or layers. Where the guide tube is laminated with stiffer layer(s) at or near the throughbore, it is preferred that the stiffer layer or layers is/are porous for the passage of air. This can allow air appearing in the throughbore to vent through the porous material of the guide tube. However, if a stiffer inner layer is not porous, air driven down the through bore may still be removed from the distal end of the guide tube when it contacts the extreme distal end of the porous outer layer. The stiffer layer(s) may be of a hydrophobic or superhydrophobic material to aid in avoiding liquid seepage through the guide tube structure. However, one or more layers may not be hydrophobic or superhydrophobic.

Thus, an inner layer, for example a layer providing the surface of the throughbore may be of a stiffer material. Examples of such a stiffer material can be an inner layer of a polymer such as polyether ether ketone (PEEK) or its copolymer forms. More generally the inner layer may be selected from the group consisting of: polyether ether ketones (PEEK); nylons; polyurethanes; polyesters; fluoropolymers such as polytetrafluoroethylene (PTFE); polymeric perfluoroethers (such as perfluoroalkoxy alkanes (PFA)) polyvinylidene difluoride (PVDF), and fluorinated ethylene propylene (FEP); Liquid Crystal Polymers (LCP); and mixtures or copolymers thereof. Such a polymer layer may be of a naturally non-porous material, but can be manufactured to be porous in various ways. For example by micro-perforating the polymer material used to form an inner layer, by drilling or by laser. For further example by weaving, braiding or electrospinning polymer fibres about a cylindrical former to form a tube of porous polymer sheet material. The use of 3D printing to produce a porous structure is also contemplated.

For example, an outer layer of ePTFE may be bonded to a relatively stiffer inner layer formed as a portion of (microperforated) PEEK tubing. The bonding of the relatively stiffer inner layer to the outer layer may be achieved by dip-coating of the inner layer, for example with an adhesive, prior to attachment of the outer layer. Advantageously the outer layer may be stretched over the inner layer before bonding. On cutting such a tube across its diameter the stretched outer layer will tend to contract at the cut end, thereby providing a 'bullet shaped' end to the tube that can be used as the distal end for insertion into the brain, with a relatively atraumatic tip profile.

To minimise trauma to the brain as far as possible the guide tube should have as small as practical outer diameter, at least when compressed and in situ in brain tissue. For example the guide tube may have an outer diameter of from 0.75mm to 2.5mm but preferably from 1.2mm to 1.7mm. Thus for example the guide tube may be 1.2mm diameter before compression on insertion into the brain and may be of approximately 1mm diameter when in situ.

For use with cannulas (micro-catheters) of the types employed to deliver infusate into brain tissue in convection enhanced delivery techniques, the throughbore of the guide tube may have a diameter of 0.4mm to 0.7mm, preferably from 0.5 mm.to 0.6mm. Advantageously the throughbore of the guide tube is sized to be a close fit to the outer surface of a cannula employed. A close fit avoids providing a route for reflux of infusate out of the brain. The cannula itself may have a diameter of 0.5 mm to 0.6mm and a throughbore of say 0.1mm to 0.4mm.

In use the guide tube has a proximal end that does not enter the brain but is fixed at or near the skull of a patient so as to secure the position of the tube in the brain. Thus, the guide tube of the invention may be provided with an enlargement at the proximal end sized and shaped for securing in a burr hole in a skull as with prior art arrangements. The enlarged proximal end will generally be made of a different material from that of the resiliently deformable outer layer of the guide tube. The two parts of different materials may be bonded together by fusion or adhesive for example.

Advantageously the guide tube may be inserted through a burr hole in the skull that has already been fitted with a guide hub. The separate guide hub constitutes a part of the neurosurgical apparatus of the first aspect of the invention and is a third aspect of the invention. The guide hub has a passage for the guide tube therethrough. The guide hub can be secured to the skull in a separate procedure and then the guide tube then inserted through the passage.

The guide hub provides a fixed datum point at the skull, from which the probes, guide tube, and cannula direction and length can be directed. Conveniently where a separate guide hub is employed, the guide tube may have an increased diameter open proximal end for securing in a guide hub in use. The increased diameter proximal end of the guide tube can sit in a corresponding shaped seat in the guide hub passage. The increased diameter proximal end may be cylindrical, part spherical or conical ('flared'), for example. The proximal end of the guide tube can then be secured to the guide hub, for example by a fitting such as a screw with a bore therethrough, which screws down onto the increased diameter proximal end of the guide tube compressing it onto the corresponding shaped seat in the guide hub. The increased diameter proximal end may be of a different material from that of the rest of the guide tube. For example, the increased diameter of a proximal end maybe formed by an overmolding process applying a different polymer onto the end of a guide tube.

Advantageously the compression of the flared proximal end of the guide tube may form a fluid and gas seal between the guide tube and the hub and between the guide tube and the screw fitting. Interposition of a deformable washer, such as a silicone washer between the distal end of the screw fitting with a central bore and the flared proximal end of the guide tube may radially compress and fix a cannula, with respect to the hub, that is passed through the bore of the screw fitting and the guide tube. In compressing the cannula such a washer can also create a fluid and gas seal between the cannula and the guide tube. A fluid and gas seal may also be created between the cannula and guide tube if the cannula has a proximal threaded stop with a male thread which is screwed onto the hub with a complimentary female thread within its bore and creates a conical seal between a conical form on the distal end of the threaded stop that is co-axial with the cannula that engages within the bore of the flared proximal end of the guide tube.

Compression of the flared proximal end of the guide tube may also form a fluid and gas seal between the guide tube and the hub and between the guide tube and the cannula/screw fitting by virtue of the resiliently-deformable outer layer of the guide tube. Although the enlarged proximal end of the guide tube may be made of a different material from that of the resiliently deformable outer layer of the guide tube, the outer layer may be extended over the flared proximal end to cover it. This can be achieved for example where an electrospun outer layer is used, by extending the region over which the outer layer is electrospun. This may avoid the need for providing one or more separate washers, which increase the complexity of assembly and the size of the seals. The outer layer may extend over the proximal flared end of the guide tube. The guide tube may be configured such that the outer layer around the proximal flared end is compressed against an interior surface of the guide hub on securing the guide tube to the guide hub.

Convection enhanced delivery techniques can be used for both acute (short time) and chronic (longer term or repeated) delivery of treatment to brain tissue. For example gene therapy may be carried out in a single treatment session, whereas treatments for other reasons such as chemotherapy may require repeated infusions into the brain (a chronic treatment regime). In either case, but especially where a chronic treatment regime is employed, it is highly desirable that the distal end of the guide tube remains in place in brain tissue. The distal end of the guide tube is typically positioned at the proximal end of a cannula trajectory as it traverses a target volume and serves to retain infusate in the target by resisting reflux at the step created between the respective diameters of the guide tube and cannula. Fixing the proximal end of the guide tube to the skull or to a guide hub that is fixed to the skull provides a relatively secure arrangement. However, the brain is moveable with respect to the skull. Thus, in use the guide tube may be subject to axial or even transverse forces when the brain moves. Such forces can result in the distal end of the guide tube repeatedly traumatising brain tissue. This may result in tissue vacuolation and the creation of a low resistance pathway at the step which will augment rather than resist reflux, causing a loss of therapy from the target.

Advantageously the guide tube is resiliently extendible and compressible in the axial direction, at least in a proximal end portion. Most advantageously the material from which the proximal portion of the guide tube is made has a Poisson's ratio which is close to zero or negative when stretched or compressed in an axial direction. Tubes made of ePTFE or polyurethane foams can for example exhibit this property, i.e. their wall thickness does not change substantially when they are compressed or extended (under small strains). More generally, many polymeric foams have a close to zero Poisson's ratio as air will tend to escape as the foam is compressed.

For example, a proximal end portion that is located, in use, outside the brain. Such a proximal end portion may extend from outside the brain to or towards the extreme proximal end of the guide tube. A guide tube that is resiliently extendible and compressible in a proximal portion can act to accept lengthening and shortening in response to changing distance between brain and a fixed proximal end of the guide tube at the skull.

Where the guide tube includes a laminated structure with a stiffer layer or layers at or near the throughbore, the stiffening may be reduced or even absent in the proximal end portion. For example a guide tube may comprise an inner tube of a stiffer material (such as perforated PEEK) overlaid with an outer layer of a porous resiliently deformable material (such as electrospun PTFE or polyurethane). The inner tube may have an over-mould on its proximal end to create a flare or stop to limit its depth of insertion and facilitate its fixation in a guide hub in the skull.

A proximal portion of the guide tube may be configured to be axially deformable. This may be achieved using a deformable portion of the inner tube that is configured to be axially deformable, for example by being formed as a spring by creating a spiral cut through the wall of the inner tube along its long axis. The deformable portion of the inner tube may have a length of between 5 and 30 mm, preferably between 10 and 20mm. With the proximal end of the guide tube fixed relative to the skull, for example by being secured in a guide hub, the deformable portion of the inner layer of the guide tube may accommodate movement of the brain relative to the skull, whilst allowing the distal end of the guide tube to remain fixed in its location in the brain.

The outer layer of the guide tube, being made of more resiliently deformable material, can be constructed to accommodate the desired range of movement over this section. For example, the outer layer may be of electro-spun, low durometer polyurethane with a high proportion of transversely aligned fibres to augment axial compliance. The outer layer may be made less adherent or non-adherent to the deformable portion of the inner layer of the guide tube to enhance compliance of the deformable portion of the guide tube. For example, electro-spun polyurethane has poor adherence to a smooth PEEK surface which can be overcome by dipping the PEEK in a polyurethane solution. If the PEEK inner tube and its over-mould are dipped in a polyurethane solution except for the deformable portion, then there will be little or no adherence of the outer layer to the deformable portion of the inner layer.

When the guide tube with the described deformable portion is delivered over a guide tube probe into the brain, the axial force applied to the guide tube may compress the deformable portion so that the guide tube's column strength is sufficient to deliver it to the desired target. In situ, with the guide tube probe removed and replaced by the cannula, the distal end of the guide tube will remain fixed at its location in the brain target even though there may be relative movement of the cannula. Relative movement of the cannula may augment reflux around the cannula. However, the reflux will be confined to the target volume because the step created by the distal end of the guide tube, that is the primary control of reflux, will be relatively fixed at its brain target.

An alternative means of constructing a guide tube with a proximal deformable portion is to provide an inner tube that extends from the distal end of the guide tube but not to the extreme proximal end. A proximal portion of the guide tube, without the inner tube, extends from the extreme proximal end of the guide tube to the beginning of the inner tube. Where the guide tube has an increased diameter proximal end, the portion of the guide tube without the inner tube may extend from a distal end of the increased diameter proximal end to the beginning of the inner tube. The increased-diameter proximal end may therefore still include an inner tube. The proximal portion can be sized to extend in use from the skull of a patient to or towards the outer layer of the brain. As a relatively stiff inner tube is absent from the proximal portion, the proximal portion can lengthen and shorten allowing the distal end of the guide tube to stay in place in the brain tissue.

In use the guide tube is inserted into the brain when mounted on a guide tube probe passing through the throughbore. To allow the guide tube probe to drive the guide tube into place it may be shaped to engage the proximal end of the guide tube, for example by having an increased diameter creating a 'step' in the outer surface of the guide tube probe that engages the extreme proximal end of the guide tube. The guide tube may be mounted on the guide tube probe using a friction fit. The guide tube probe may be configured such that the friction between the guide tube probe and the guide tube is less than the friction between the guide tube and the guide hub once the guide tube is secured to the guide hub. Thereby, the guide tube probe can be easily withdrawn from the through-bore of the guide tube once the guide tube has been inserted to the intended position and secured to the guide hub.

Where the guide tube has a proximal portion without the inner tube, as discussed above, a guide tube probe employed may have two steps. One engages the top of the inner tube to apply an insertion force, the other engages the extreme end of the proximal portion and can be positioned to compress the proximal portion, typically only slightly e.g., by 1-3mm if the proximal portion is about 1 to 1.5 cm long. This compression on insertion provides a slight preload to the proximal portion, so it can lengthen readily if the skull to brain distance lengthens.

As mentioned above, it may be necessary to cut the guide tube to a desired length prior to implantation of the guide tube into the brain. The resiliently deformable outer layer may make cutting of the guide tube more difficult, because the outer surface of the guide tube will tend to deform when a cutting force is applied. To address this, the guide tube may be provided as part of a package. The package may comprise the guide tube and a packaging tube, wherein the guide tube is provided within the packaging tube. The packaging tube may have a stiffness that is greater than that of the outer layer of the guide tube. The packaging tube holds the guide tube in place and reduces its tendancy to deform when a cutting force is applied, ensuring a clean axial cut of the distal end of the guide tube. Once the guide tube has been cut to length, it can be removed from the package immediately prior to its insertion into the brain. The packaging tube also reduces direct handling of the outer surface of the guide tube before implantation, thereby reducing the chance of contamination of the guide tube, for example with pathogens. The package may further comprise a stylet within the through-bore of the guide tube. The stylet further reduces deformation of the guide tube during cutting.

The packaging tube may be configured to compress the outer layer of the guide tube while the guide tube is within the packaging tube. This aids in preventing lateral deformation of the guide tube during cutting, but also allows an axial shear force to be applied to the outer layer of the guide tube by distal displacement of the packaging tube relative to the guide tube. If, prior to cutting the guide tube, the packaging tube is moved distally along the axis of the guide tube to impose shear forces on its outer layers that move them distally, a bullet shaped distal end of the guide tube may be achieved by the axial cut of the guide tube. When the packaging tube is removed from the guide tube, proximal retraction of the outer layers once the shear force is removed will result in a bullet shaped distal profile.

As mentioned above, the guide tube is inserted into the brain down a premade track. The premade track is conventionally made with a round tipped relatively rigid probe. However, the use of such a probe can present practical difficulties. A probe inserted into the brain has to first pass through the pia, the tough outer membrane covering the cortex. This is not easily achieved with a blunt tip which will tend to displace the brain before it traumatically breaches the cortical surface. As a blunt probe passes to its target the brain will tend to deform around the probe depending on the compliance of the tissue it is passing through. This in turn depends on whether it is grey or white matter and the orientation of the white matter tracts of an individual's brain. As result of the brains deformation during the insertion of a probe, the probe can deviate from the planned trajectory leading to a targeting error.

According to a fourth aspect the present invention provides a probe for insertion into tissue comprising a rod having a rounded or conical distal end provided with an axially extending, narrower diameter spike having an extreme end for dissecting tissue. The axially projecting narrower diameter spike may be provided with a rounded extreme end. The axially projecting narrower diameter spike may taper from the rounded distal end of the rod to the extreme end of the spike. The probe can be used for insertion of cannulas into brain tissue but other uses and use in other tissue types is also contemplated. For example, when inserting of DBS (Deep Brain Stimulation) or recording electrodes. For each surgical indication the diameter of the probe can be selected to create a track that is sized be of the same diameter or just less than that of the device to be inserted. DBS electrodes leads typically have an outside diameter (OD) of 1.3 mm and so in this instance a probe with an OD of 1.3mm or less, e.g.1.2 mm, may be used. In the latter case the rounded end of the DBS electrode lead would dilate the track created by the 1.2mm diameter probe as it is inserted. The probe may be coated with a biocompatible lubricious material (for example Parylene or PTFE). This reduces shear forces on surrounding tissue when the probe is inserted, and thereby causes less micro trauma to the brain tissue.

The probe can be used in preparing a track (in brain tissue) for the guide tube of the first aspect of the invention. The spike on the probe may typically be the same or a slightly smaller diameter than the cannula being employed. Thus, the probe may be provided as part of a kit for convection enhanced delivery of an infusate to the brain parenchyma, that constitutes a fifth aspect of the invention. The kit may include a probe for insertion into tissue to create a track for a cannula, for example as discussed immediately above, a guide tube, an associated guide tube probe and, where the guide tube is not fitted with an enlarged proximal end for fitting to a burr hole, a guide hub.

According to a further aspect, the invention provides a cannula for insertion through a guide tube into the brain, to deliver an infusate to a target brain volume. The cannula may be provided as part of the above-mentioned kit for convection enhanced delivery of an infusate to the brain parenchyma. Once the cannula is fitted through the guide tube, it can be used for delivery of infusate to the brain. It is important that air not be delivered into the brain through the cannula, and to reduce this risk, the cannula may comprise a bubble vent. The bubble vent is preferably provided at a proximal end of the cannula. The bubble vent is configured to prevent gas from entering the cannula. The bubble vent mitigates the risk of bubbles entering the brain if they have come out of solution in the infusate or have been entrained in the infusate during the connection and/or disconnection of a delivery system that is used to deliver the infusate to the cannula, such as a dispenser, infusion line, and/or pump. Bubbles infused into brain tissue can tear the tissue and disrupt distribution of the therapeutic fluid/infusate. The bubble vent may be permanently joined to and/or integrally formed with the cannula, which further reduces the chance of bubbles being entrained during connection of the cannula to the delivery system. The bubble vent may additionally or alternatively be configured to prevent pathogens (for example micro-organisms such as bacteria) from entering the cannula. This reduces the risk of intracranial infections occurring due to the treatment.

The bubble vent may comprise a low volume bubble filter, for example made from expanded polytetrafluoroethylene (ePTFE). The bubble filter may have a hydrophobic (optionally superhydrophobic), gas permeable, microporous structure configured to remove bubbles from the flowing therapeutic fluid/infusate. The bubble filter may be effective to remove bubbles at flow rates of less than or equal to 30 µl/min. The bubble vent may further comprise a filter guard. The filter guard may comprise a distribution of a plurality of perforations (small holes) that facilitate degassing the fluid/infusate as it flows through the bubble vent to the cannula. The filter guard may also guard/protect the bubble filter against damage. The combination of the bubble filter and the filter guard facilitates dispersion of entrained air/bubbles from the fluid flow before the fluid passes into the cannula.

The bubble vent may comprise a retaining cap, which connects with the filter guard to complete the assembly of the bubble vent and to contain the bubble filter within the bubble vent. The retaining cap may include a septum stopper, which provides a sealed unit until the septum is pierced by a hollow needle to provide fluid connection to the cannula. The septum stopper may be retained under compression by a septum cap. The retaining cap and filter guard may be joined by a snap fit connection. However, alternative arrangements could be used to join them together, for example a threaded connection, welded connection, glued connection etc.

The bubble vent incorporating the bubble filter reduces the risk of air being delivered e.g. to the brain with the fluid containing therapeutic agent/infusate. It will be appreciated fluid containing air/bubbles will be space occupying and therefore is capable of stretching and tearing brain tissue whilst also disrupting delivery/distribution of the therapeutic agent/infusate. The bubble vent can also act to filter out pathogens, including bacteria and other microorganisms from the fluid.

In use, the spiked end of the probe can dissect tissue with reduced trauma as it is inserted, creating a reduced resistance pathway for the rounded or conical end which dilates the tissue to the larger diameter of the rod. For creating a track for use with the guide tubes of the invention the probe may have a diameter of 1.2mm or less. The spike may be 4 to 5mm long and may have a taper from 0.6 or 0.5 mm to 0.3mm or 0.2mm at its extreme distal end. For example, the spike may have a taper from 0.5mm down to 0.3mm at its extreme distal end. Where the probe has a 1.2mm outer diameter the track produced in brain tissue will tend to be of a slightly smaller diameter, for example 1.1mm after removal of the probe. The probe may be made from hardened stainless steel or tungsten carbide. In addition to providing a means for providing a track for the guide tube of the invention, the spike of the probe can provide a proximal track for a cannula inserted into tissue beyond the end of the guide tube in the usual way.

The present invention also provides methods of surgery making use of the apparatus described herein.

Thus, according to a sixth aspect, the present invention provides a surgical method for convection enhanced delivery of an infusate to the brain parenchyma, the method comprising:
a) passing a guide tube into the brain parenchyma, wherein the guide tube comprises:
   a proximal end;
   a distal end; and
   a through-bore for passage of a cannula;
   wherein at least an outer layer the guide tube is of a hydrophobic material that is resiliently deformable and porous to allow passage of air; and
   wherein the guide tube is passed into the brain with the aid of a guide tube probe passing through the throughbore so that its distal end is at or just beyond the distal end of the guide tube;
b) when the distal end of the guide tube is at its planned position, advancing the guide tube probe further along the trajectory to create a track through the brain tissue to accommodate the cannula;
c) removing the guide tube probe;
d) passing a cannula through the throughbore and into the brain along the track; and
e) passing an infusate into the brain via the cannula.

As an alternative to steps b) and c) in the method described above, the creation of the track for the cannula may be created by:
b1) when the distal end of the guide tube is at its planned position, removing the guide tube probe;
b2) inserting a track making probe down the guide tube along the trajectory to create a track through the brain tissue to accommodate the cannula;
c1) removing the track making probe; and

then carrying out steps d) and e).
he track making probe employed in step b2) may be a probe for insertion into tissue in accordance with the fourth aspect of the invention, as described herein.

More generally, each one of the guide tube, guide tube probe, probe for creating a track for the cannula and the cannula may include any of the features described herein with respect to the other aspects of the invention.

The infusate may carry any suitable therapeutic agent, imaging agent or diagnostic agent that can be delivered into the brain tissue via a suitable biologically inert fluid.

### BRIEF DESCRIPTION OF THE DRAWINGS

Embodiments of the invention will now be described with reference to the following schematic drawings, in which:
Fig. 1 shows a prior art neurosurgical assembly with reflux;
Fig. 1a is a magnified view of part of figure 1;
Fig. 2a shows a magnified view of part of a neurosurgical assembly according to the invention;
Fig. 2b shows a cross section of a guide tube as depicted in figure 2a;
Figs. 3a and 3b show cross section views of shows part of a neurosurgical assembly according to the invention;
Figure 3c shows a neurosurgical assembly in use;
Figures 4a and 4b show the ends of probes for creating a track in brain tissue;
Figure 5 shows a bubble vent attached to the proximal end of the cannula;
Figure 6 shows an exploded view of the bubble vent;
Figure 7 shows an alternative design of bubble vent;
Figure 8 shows an exploded view of the bubble vent of Figure 7;
Figure 9 shows a cross section of the bubble vent of Figures 7 and 8;
Figure 10 shows a close up view of the bubble vent of Figure 9 in use;
Figure 11 shows a cross-section of a guide tube having an outermost layer;
Figure 12 shows components of a kit for convention enhanced delivery of an infusate;
Figure 13 shows an assembled neurosurgical apparatus;
Figure 14 shows a guide tube with a perforated inner tube extending through substantially its entire length;
Figure 15 shows a close-up view of the proximal end of an assembled neurosurgical apparatus.

### DETAILED DESCRIPTION OF THE DRAWINGS

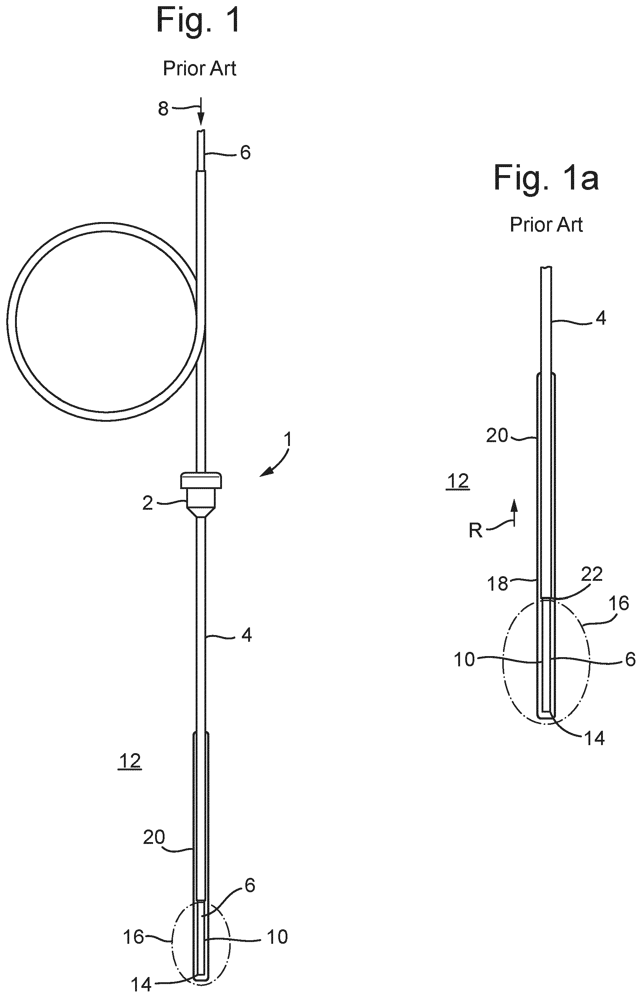

Fig 1 shows a prior art neurosurgical assembly 1 comprising an enlarged head 2, at the proximal end of a guide tube 4 and a cannula 6 that extends from a source of infusate 8 through the guide tube 4 to a distal end 10. The enlarged head 2 is secured in a burr hole (not shown) in the skull of a patient and the distal end of the cannula 10 is positioned within brain tissue 12 so as to deliver infusate from the extreme distal end 14 of the cannula 6 into a target volume of brain tissue as suggested by dashed line ellipse 16.

However, and as indicated in figure 1, the infusate may reflux, finding a path of lower resistance along the outside wall of cannula 6 and guide tube 4, in an axial flow 20 that extends (magnified view figure 1a) from the extreme end 14 of cannula 6 up the outside of the distal end. Magnified view figure 1a shows the axial flow 20 along the distal ends 10 and 18 of cannula 6 and guide tube 4, resulting in loss of infusate from the region of target volume 16. The step (abrupt change in diameter) caused by the change in diameter from the cannula 6 to the, square cut, extreme end distal end 22 of the guide tube 4 can act against reflux continuing in the direction suggested by arrow R. However, the step can be overwhelmed by fluid volume and the prevention of reflux along the outside surface of the guide tube 4 can depend on the extent of sealing interaction between the guide tube 4 and brain tissue 12. That sealing interaction may not be sufficient to prevent fluid passing the step at distal end 22 of guide tube 4.

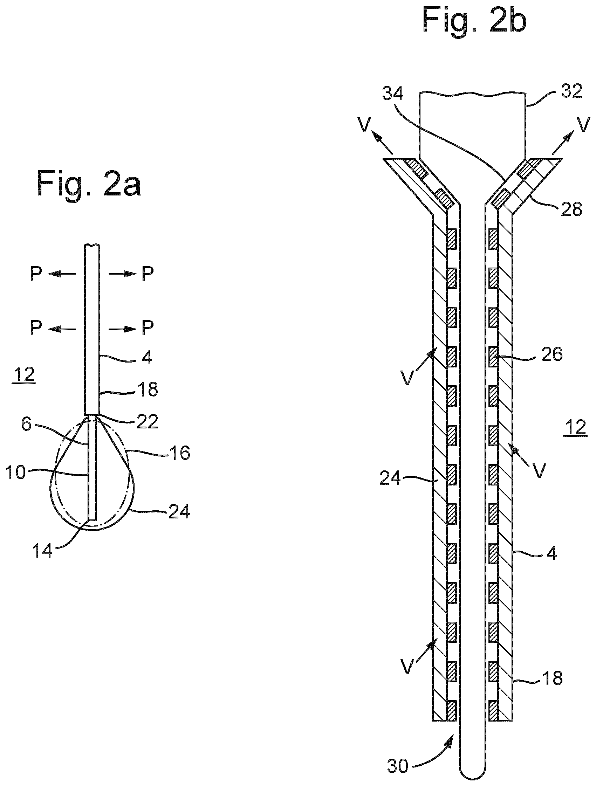

Figure 2a shows in magnified view the distal ends 18 and 14 of the guide tube 4 and cannula 6, where the guide tube 4 is in accordance with the invention. The intended result of an infusion is to deliver infusate into the target volume 16 of brain tissue 12. In practice, successful delivery of infusate may approximate to that suggested by 'teardrop' shape 24.

In the example of figure 2a the guide tube 4 is resiliently deformable, hydrophobic or superhydrophobic and porous to air. On insertion into a premade track made in brain tissue 12, the guide tube 4 has been compressed radially inwards. In place as depicted, the guide tube 4 exerts pressure radially outwards in attempting to return to its natural (uncompressed) state. This pressure (suggested by arrows P) provides at least an initial sealing interaction with the brain tissue 12. Thus, the guide tube of the invention exerts pressure along its length to aid in preventing reflux.

In addition, the porous nature of at least the outer layer of guide tube 4 can allow integration with brain tissue 12 over time, blocking the reflux path.

Figure 2b shows the whole length of the guide tube of figure 2a in schematic cross section (not to scale). In this example the guide tube has an outer layer 24 of ePTFE and an inner layer 26 of porous (microperforated) PEEK. The guide tube 4 has a flared proximal end 28 for seating in a guide hub as described hereafter. Through bore 30 is for passage of a cannula to deliver infusate. When the guide tube is being inserted into the brain the throughbore 30 is fitted with a probe 32 as shown in the figure. Probe 32 incudes a step 34, a change in diameter, to correspond to and engage with flared end 28 of the guide tube 4.

The outer layer 24 is porous and superhydrophobic. Therefore, the outer layer is porous to air but not to aqueous fluids. The inner layer 26 of microperforated PEEK is relatively hydrophobic with a water contact angle between 70° and 90°. When in place or being driven into brain tissue 12, air present outside inner layer 4 or in throughbore 30 will tend to enter the body of the guide tube and vent to atmosphere as suggested by arrows V.

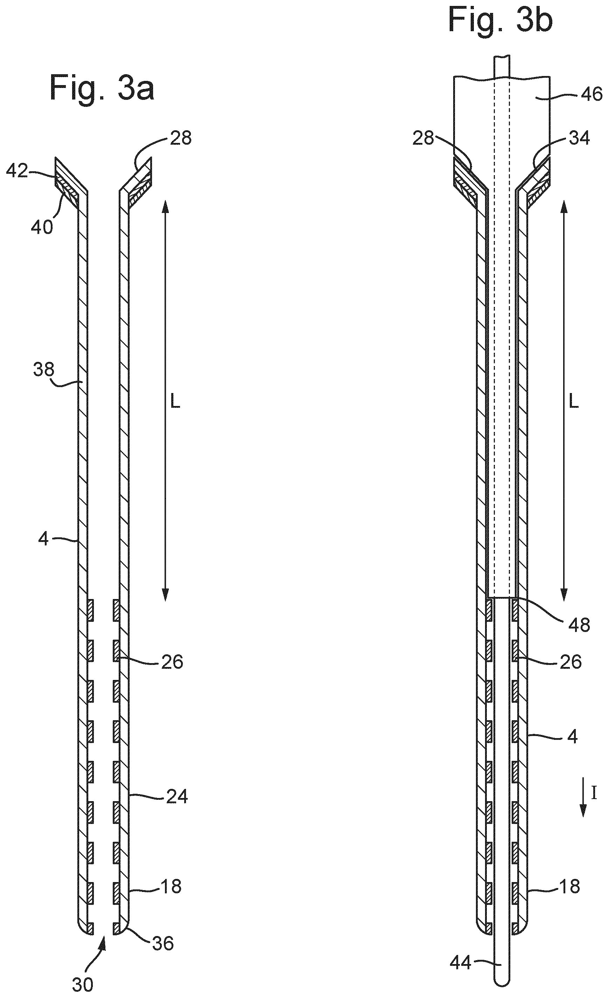

Figure 3a shows an alternative guide tube to that of figure 2b in schematic cross section, with like parts numbered the same. In this example the lower part of outer layer 24, including the distal end 18 has been bonded to the inner layer of perforated PEEK 26 after stretching the (ePTFE) material. Consequently, the extreme distal end 36 of the outer layer 24 is under tension and deforms to a rounded bullet like shape. This may aid in avoiding trauma on insertion into brain tissue. The bullet-like shape may also be provided by cutting the end of the guide tube while inside a package, as described above.

In this example the inner perforated PEEK layer does not extend the whole length of the guide tube 4. An upper portion (proximal portion 38) of the guide tube 4 is not lined. This portion of the guide tube is resiliently extendible and compressible in the axial direction. The unlined proximal portion has a length L. This length may be relatively short, for example of the order of 1 to 1.5cm, designed in use to span the distance from a patient's skull to the surface of the brain.

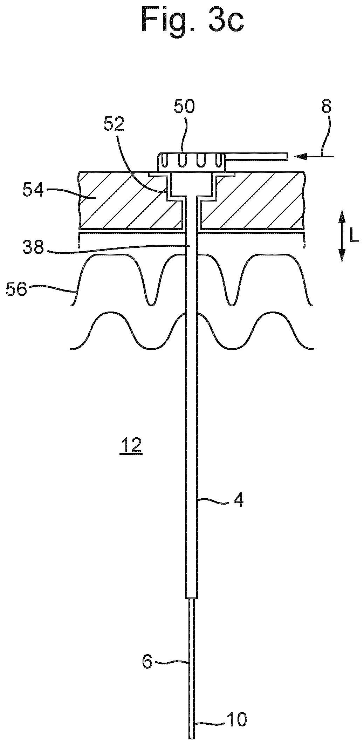

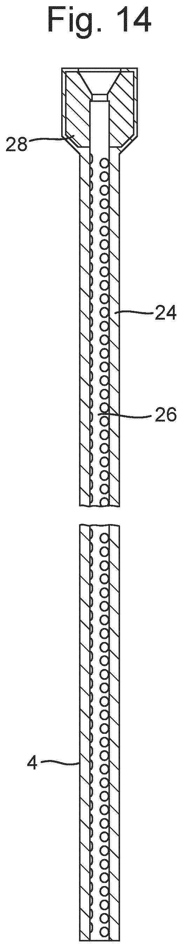

In use as described below with reference to figures 3b and 3c the proximal portion 38 may be compressed on insertion into a patient to then act as a shock absorbing element, capable of accommodating relative motion between brain and skull. As the proximal portion 38 is formed of ePTFE in this example it may show a change in density but little change in diameter on stretching or compressing. Alternatively, the inner perforated layer 26 may extend over substantially the entire length of the guide tube 4, as shown in Fig. 14.

Also shown in figure 3a are an ePTFE washer 40 and a PEEK washer 42 to aid sealing to a seat in a guide hub (not shown in this figure). The washers 40, 42 may also help to create a fluid and gas seal between the guide hub and the guide tube 4.

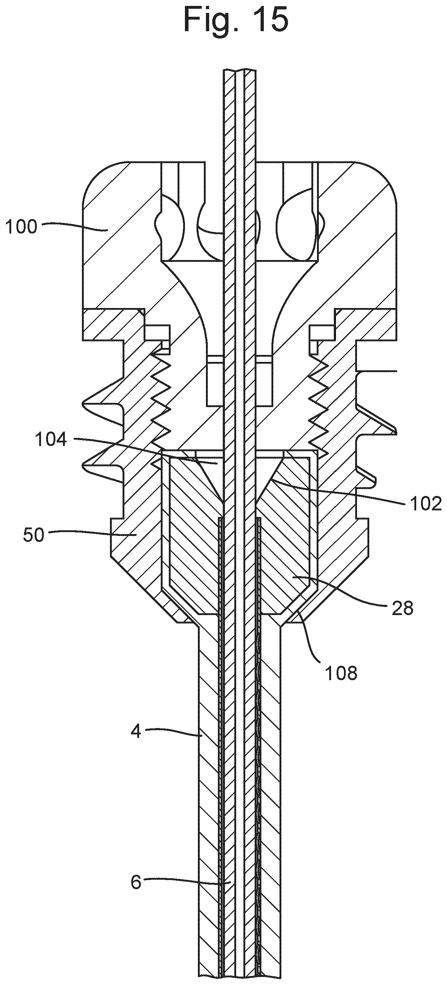

Fig. 15 shows a close-up view of the proximal end of the guide tube 4 during use, when engaged with a guide hub 50, cannula 6 and cap 100. In this example, no washers are present. A fluid and gas seal between the guide tube 4 and the guide hub 50 and between the guide tube 4 and the cannula 6 is created through compression of the resiliently-deformable outer layer of the flared proximal end 28 of the guide tube 4. Compression of the outer layer of the guide tube 4 creates a proximal seal 102 with a stop 104 provided on the cannula 6. Compression of the outer layer of the guide tube 4 also creates a distal seal 108 with the guide hub 50.

Figure 3b shows the arrangement of figure 3a but being inserted into the brain as suggested by arrow I. insertion is carried out with the aid of two coaxially arranged probes. A central probe 44 passes through the through bore 30 and extends below the distal portion 18 of guide tube 4. An outer probe 46 fits around central probe 44 and has a step 34 for engaging with flared guide tube end 28. Outer probe 46 also has a step 48 for engaging with the upper end of liner tube 26. When inserting the guide tube 4, as suggested by arrow I, the length L of the unlined proximal portion of guide tube 4 is shortened (compressed) as the distance between steps 34 and 48 is less than the uncompressed length L. As an alternative to the dual probe arrangement shown a single probe including both steps 34 and 48 in its outer profile and passing down through the whole length of throughbore 30 could be employed.

Figure 3c shows the guide tube 4 of figures 3a and 3b in use. The guide tube 4 is seated in place in a guide hub 50, fitted in a burr hole 52 of a patient's skull 54. The guide tube has been inserted through the pia 56 of a patient's brain tissue 12. Cannula 6 fed with infusate fluid from a source 8 passes down the guide tube throughbore with a distal end 10 extending beyond the guide tube 4. The unlined proximal portion 38 of the guide tube 4 is compressed as discussed above with respect to figure 3b. If the brain tissue 12 moves relative to the skull 54 the proximal portion 38 can move resiliently to accommodate the displacement, avoiding disturbance to the guide tube 4 where it is inside brain tissue 12. For example, the uncompressed length L of unlined proximal portion 38 may be 1.3cm with the length l compressed to 1cm on fitting in place.

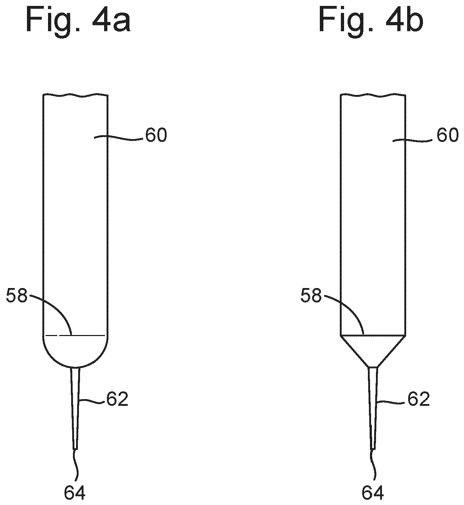

Figure 4a shows the end 58 of a hardened stainless steel probe 60. The end 58 is rounded ('bullet nosed') in shape and has a projecting spike 62 in the form of a rod tapering from rounded end 58 to the extreme distal end 64 of the spike 62. An alternative end 58 with a conical shape is shown in figure 4b. In both examples, the extreme end 64 of the spike is itself rounded. The probes find use in dissecting tissue to form a track, for example to form a track through brain tissue prior to inserting guide tubes such as those discussed above and shown in figures 1 to 3.

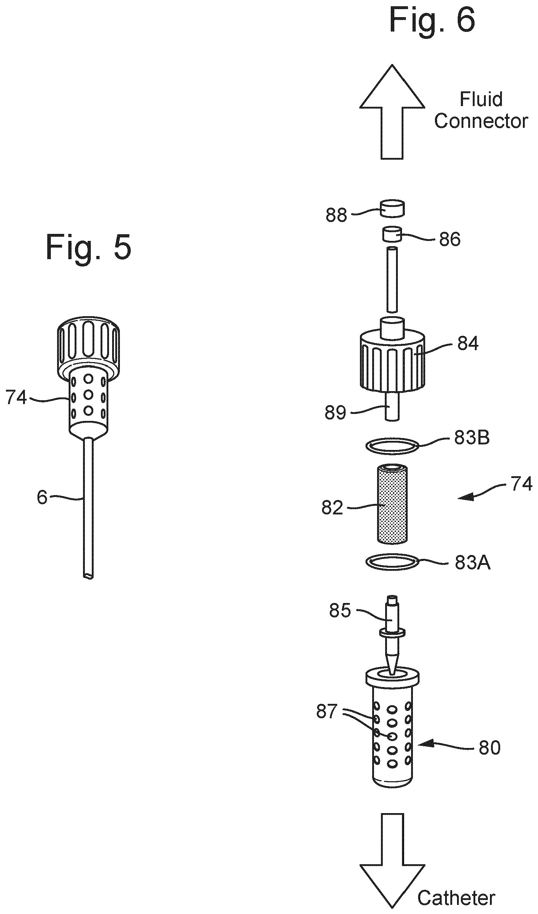

Fig. 5 shows a proximal end of a cannula 6 comprising a bubble vent 74 provided at the proximal end of the cannula 6. The configuration and component parts of the bubble vent 74 are described below and illustrated in Fig. 6. In summary, the bubble vent 74 mitigates the risk of bubbles entering the brain if they have come out of solution in the infusate or have been entrained in the infusate during the connection and/or disconnection of a delivery system that is used to deliver the infusate to the cannula 6, such as a dispenser, infusion line, and/or pump. The bubble vent 74 is preferably provided integrally with the cannula 6, as shown in Fig. 5, which further reduces the chance of bubbles being entrained during connection of the cannula 6 to the delivery system. The bubble vent 74 may also be configured to prevent pathogens (for example micro-organisms such as bacteria) from entering the cannula 6.

Fig. 6 illustrates an exploded view of the bubble vent 74, configured for attachment to the cannula 6 and a fluid connector of the delivery system. The bubble vent 74 includes a perforated filter guard 80, a bubble filter 82, retaining rings 83A, 83B, a retaining cap 84, a septum stopper 86 and a septum cap 88.

In the illustrated example, the bubble filter 82 is a low volume bubble filter made from expanded polytetrafluoroethylene (ePTFE), which has a super-hydrophobic, gas permeable, microporous structure configured to remove bubbles from the flowing therapeutic fluid. In the illustrated example the bubble filter 82 is effective to remove bubbles at flow rates of less than or equal to 30 µl/min. The filter 82 is contained in the perforated filter guard 80 and in the illustrated example the bubble filter 82 is received over a hollow post 85 and is retained by a retainer ring 83A to the hollow post 85, which is positioned concentric to the filter guard 80.

In the illustrated example, the filter guard 80 is a hollow shell that includes a distribution of a plurality of perforations (small holes) 87 around the shell wall. The perforations 87 facilitate degassing the fluid as it flows through the bubble filter 82 from the delivery system to the cannula 6. The filter guard 80, as the name suggests also guards/protects the filter 82 against damage. The combination of the bubble filter 82 and the filter guard 80 facilitates dispersion of entrained air/bubbles from the dispenser fluid flow before the fluid passes into the cannula 6.

The bubble vent 74 also includes a retaining cap 84, which connects with the filter guard 80 to complete the assembly of the bubble vent 74 and to contain the bubble filter 82 within the bubble vent 74. The retaining cap 84 includes a hollow retaining post 89 and a retainer ring 83B which engage with the bubble filter 82 to ensure the bubble filter 82 is correctly positioned and retained in the filter guard 80 to ensure efficient functionality of the bubble filter 82 during use.

In the illustrated example, the retaining cap 84 includes a septum stopper 86, which provides a sealed unit until the septum 86 is pierced by a hollow needle to provide fluid connection to the cannula 6. The septum stopper 86 is retained under compression by the septum cap 88.

In the illustrated example, the retaining cap 84 and filter guard 80 are joined by a snap fit connection. However, alternative arrangements could be used to join them together, for example a threaded connection, welded connection, glued connection etc.

The bubble vent 74 incorporating the low volume bubble filter 82, reduces the risk of air being delivered e.g. to the brain with the fluid containing therapeutic agent/infusate. It will be appreciated fluid containing air/bubbles will be space occupying and therefore is capable of stretching and tearing brain tissue whilst also disrupting delivery/distribution of the therapeutic agent/infusate. The bubble vent 74 can also act to filter out pathogens, including bacteria and other microorganisms from the fluid.

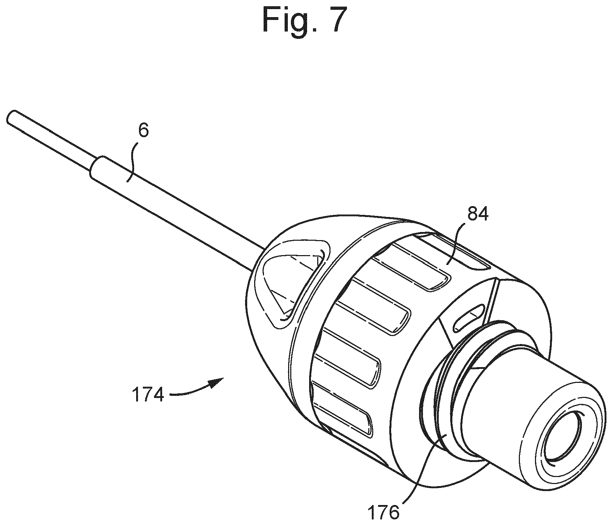

Figs. 7 to 10 show an alternative design of bubble vent 174. Fig. 7 shows the bubble vent 174 in its assembled state ready for use. Similar to the bubble vent 74, the bubble vent 174 comprises a retaining cap 84. The retaining cap 84 comprises a proximal connector 176, for example a threaded connector, for connection to a fluid connector of the delivery system.

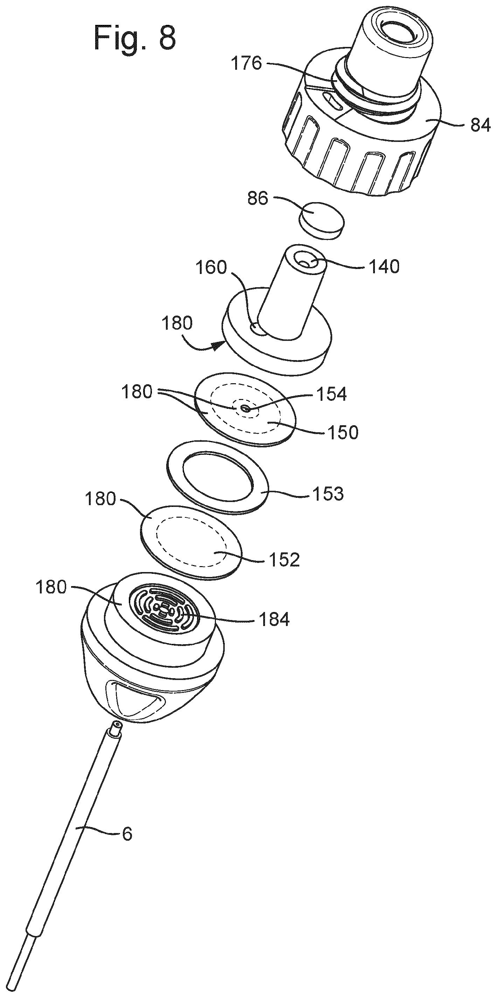

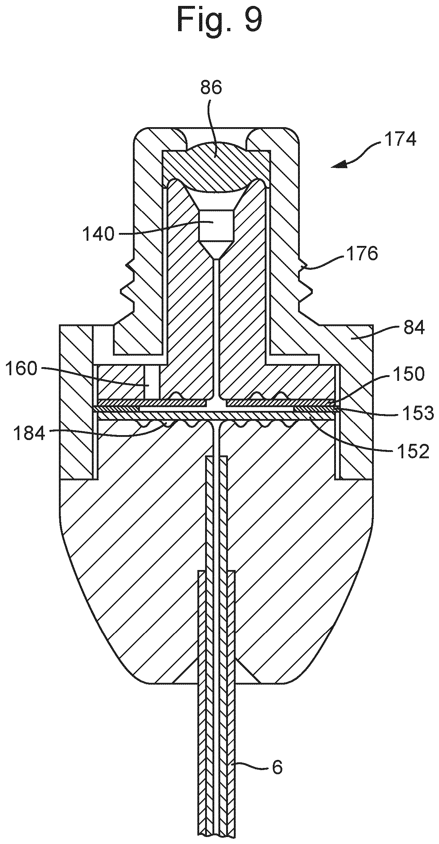

Fig. 8 shows an exploded view of the bubble vent 174, and Fig. 9 shows a cross-sectional view. The proximal connector 176 may comprise a septum 86 to seal the proximal connector 176 until the septum 86 is pierced, for example by a hollow needle. The bubble vent 174 comprises a fluid passage 140 fluidly connecting the proximal connector 176 and the cannula 6. In this design, the bubble vent comprises a first membrane 150 and a second membrane 152. The first membrane 150 and the second membrane 152 are positioned between a distal end of the fluid passage 140 and a proximal end of the cannula 6. The first membrane 150 and the second membrane 152 may be substantially parallel to one another, and may be substantially perpendicular to an axis of the fluid passage 140. The first membrane 150 is positioned closer to the distal end of the fluid passage 140 than the second membrane 152, such that fluid entering the bubble vent 174 through the septum 86 reaches the first membrane 150 before the second membrane 152. An annular washer 153 may be positioned between the first membrane 150 and the second membrane 152 that forms a peripheral fluid seal between the membranes and the housing of the connector 174 and separates the membranes centrally to create a cylindrical gap between them. The cylindrical gap may have a diameter of between 2mm and 6mm but is most preferably 4mm. The gap may separate the membranes 150 and 152 by 0.05mm to 0.2mm but most preferably by 0.1mm. The first membrane 150 and the second membrane 152 may be connected to one another and to the other components of the bubble vent 174 via connection surfaces 180. The connection surfaces 180 may be joined by any suitable method, for example using ultrasonic welding or using an adhesive layer. The bubble vent 174 may comprise a support member 184 to support the distal surface of the second membrane 152 and allow liquid that has passed through the second membrane 152 to more easily reach the cannula 70.

The first membrane 150 is hydrophobic and gas permeable. A hole 154 is provided in the first membrane 150 where the fluid passage 140 meets the first membrane 150, such that fluid from the fluid passage 140 can pass through the first membrane 150 via the hole 154. The septum sealed connector 174 may comprise a support member to support the proximal surface of the first membrane 150 and annular connection surfaces to attach the membrane around its periphery and around its central hole 154 (not shown in Fig. 8). The second membrane 152 is liquid permeable and preferably hydrophilic. It is not essential that the second membrane 152 is hydrophilic, however gas venting works most efficiently using the combination of a hydrophobic and a hydrophilic membrane. Use of the hydrophobic first membrane 150 alone could result in air being drawn from the atmosphere through the first membrane 150 and into the infusate if the pressure in the line falls below atmospheric pressure. This can happen if the connector is elevated above the head by more than 10-25 cm (depending on intracranial pressure). The second membrane 152 being hydrophilic prevents air ingress into the brain even in such situations. The second membrane 152 is impermeable to gas and bacteria. No hole is provided in the second membrane 152, such that fluid from the fluid passage 140 must pass through the material of the second membrane 152 to reach the cannula 6. One or more vent holes 160 (for example, two vent holes in the example of Fig. 7) are provided in the bubble vent 174 on a proximal side of the first membrane 150. No holes are provided in the first membrane 150 where the vent holes 160 meet the first membrane 150, such that fluid from the fluid passage 140 must pass through the material of the first membrane 150 to reach the vent holes 160.

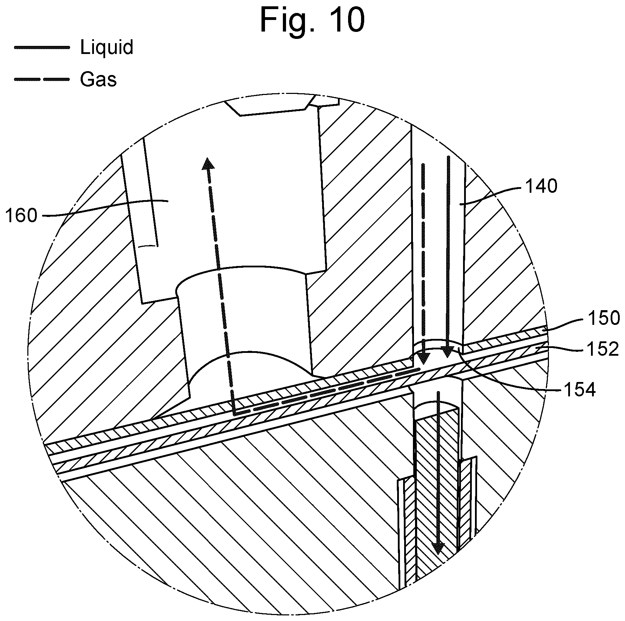

The operation of the bubble vent 174 is demonstrated in the close-up view of Fig. 10. A mixture of liquid and gas (for example an infusate to be delivered to a patient's brain via the cannula 6 with some entrained bubbles of air) enters the bubble vent 174 via the septum 86 and the fluid passage 140. The mixture passes through the first membrane 150 via the hole 154. The liquid is drawn to the hydrophilic second membrane 152, and soaks through the second membrane 152 (which is liquid permeable) into the cannula. The layer of liquid and the second membrane 152 form a barrier preventing gas from passing into the cannula 6. The gas will pass along the air gap between the first membrane 150 and the second membrane 152, and can escape through the gas-permeable first membrane 150 at the position of one of the vents 160. The hydrophobic nature of the first membrane 150 repels liquid and prevents the liquid forming a similar barrier as on the second membrane 152, thereby allowing the gas to pass out of the bubble vent 174 via the vent holes 160.

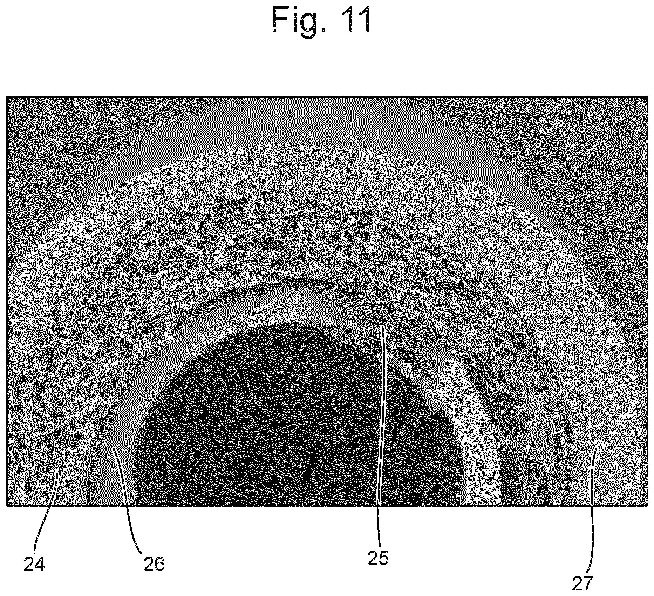

Fig. 11 shows a cross-sectional view perpendicular to the long axis in an example in which the guide tube 4 comprises an outermost layer 27 axially outward of the outer layer 24, wherein the outermost layer 27 comprises a hydrophilic material. The outermost layer 27 of hydrophilic material may contribute to improving the lubricity and/or tissue integration properties of the guide tube 4, as described above. In this example, the inner layer is formed of PEEK with perforations 25, the outer layer 24 is formed of electrospun hydrophobic polyurethane, and the outermost layer 27 is formed of hydrophilic polyurethane.

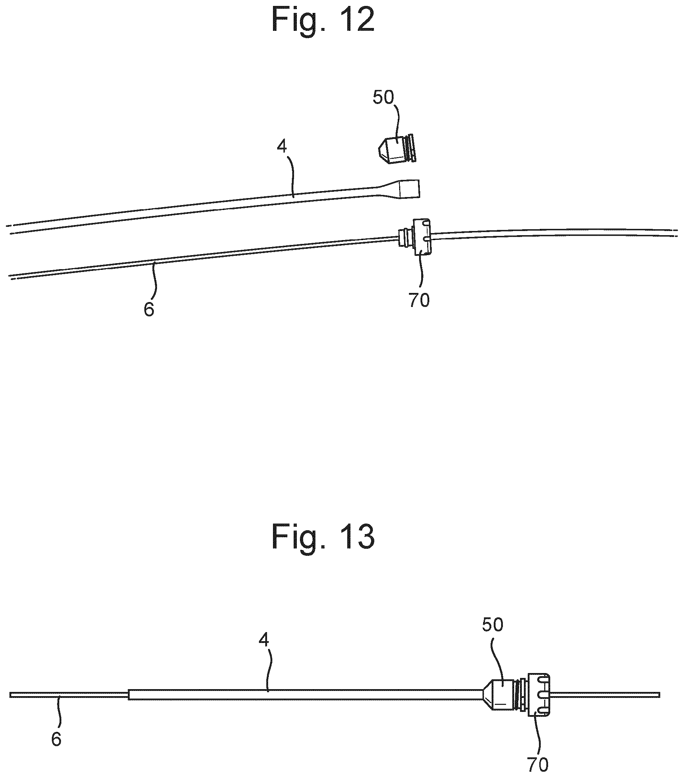

Fig. 12 shows components of a kit for convection enhanced delivery of an infusate to the brain parenchyma. The components include the guide hub 50, the guide tube 4, the cannula 6, and the threaded stop 70 which is screwed into the guide hub 50.

Fig. 13 shows the assembled neurosurgical apparatus, with the cannula 6 passed into the through-bore of the guide tube 4, and the threaded stop 70 screwed into the guide hub 50.

This application is a divisional of EP 22721111.7. The following are the claims of the parent application EP 22721111.7, and not the claims of this divisional application. However, the claims of the parent application form part of the content of this divisional application, and the applicant reserves the right to pursue protection for the subject matter defined by the claims of the parent application. The claims of this divisional application follow under the heading CLAIMS below.
1. A neurosurgical apparatus for convection enhanced delivery of an infusate to the brain parenchyma, the apparatus comprising: a guide tube for insertion into the brain and having a proximal end, a distal end and a through-bore for passage of a cannula; wherein at least an outer layer of the guide tube is of a hydrophobic material that is resiliently deformable and porous to allow passage of air.
2. The neurosurgical apparatus of claim 1, wherein at least the outer layer of the guide tube is superhydrophobic.
3. The neurosurgical apparatus of claim 1 or claim 2, wherein at least the outer layer of the guide tube comprises at least one of ePTFE, silicone foam, polyurethane foam, shape memory polymer, polymers extruded as microporous hollow fibres and electrospun polymers.
4. The neurosurgical apparatus of claim 3 wherein at least the outer layer of the guide tube comprises at least one polymer extruded as microporous hollow fibres, or an electrospun polymer:wherein the polymer extruded as microporous hollow fibres, or electrospun polymer is selected from the group consisting of; PTFE (polytetrafluoroethylene), PVDF (polyvinylidene difluoride), PU (polyurethane), polypropylene, or mixtures and/or copolymers thereof.
5. The neurosurgical apparatus of any preceding claim, wherein the guide tube further comprises an outermost layer axially outward of the outer layer, wherein the outermost layer comprises a hydrophilic material.
6. The neurosurgical apparatus of claim 5, wherein the outermost layer comprises a mixture of hydrophilic and hydrophobic materials.
7. The neurosurgical apparatus of any of claims 1-6, wherein the outer layer or outermost layer comprises a coating and/or surface treatment configured to improve lubricity and/or to promote tissue integration, optionally wherein the coating comprises a hydrophilic material.
8. The neurosurgical apparatus of any preceding claim, wherein at least the outer layer of the guide tube has a Poisson's ratio of zero or less.
9. The neurosurgical apparatus of any preceding claim, wherein the guide tube is constructed of a non-homogeneous material and/or of a plurality of materials with different stiffnesses.
10. The neurosurgical apparatus of claim 9, wherein the guide tube is constructed of a foam having increasing density from the outside radially inwards towards the throughbore, or a region of increased density at or near the throughbore.
11. The neurosurgical apparatus of claim 9, wherein the guide tube has a laminated structure with a stiffer layer or layers at or near the throughbore.
12. The neurosurgical apparatus of claim 11, wherein the stiffer layer or layers are porous to air.
13. The neurosurgical apparatus of claim 11, wherein a stiffer layer forms the surface of the throughbore.
14. The neurosurgical apparatus of any one of claims 11 to 13 wherein at least one of the stiffer layer or layers comprises a polymer selected from the group consisting of: polyether ether ketones (PEEK); nylons; polyurethanes; polyesters; fluoropolymers such as polytetrafluoroethylene (PTFE), polymeric perfluoroethers such as perfluoroalkoxy alkanes (PFA), polyvinylidene difluoride (PVDF), and fluorinated ethylene propylene (FEP); liquid crystal polymers (LCP); and mixtures or copolymers thereof.
15. The neurosurgical apparatus of any one of claims 11 to 14 wherein at least one of the stiffer layer or layers has been manufactured by a process comprising at least one of: micro-perforating sheet material of a polymer film by drilling or by laser; weaving, braiding or electrospinning polymer fibres about a cylindrical former to form a tube of porous polymer sheet material; and 3D printing a polymer in a porous form.
16. The neurosurgical apparatus of any preceding claim, wherein the guide tube has an outer diameter between 0.75mm to 2.5mm.
17. The neurosurgical apparatus of any preceding claim, wherein the throughbore of the guide tube has a diameter of from 0.4mm to 0.7mm.
18. The neurosurgical apparatus of any preceding claim, further comprising a guide hub for securing to the skull of a patient before insertion of the guide tube and having a passage for the guide tube therethrough.
19. The neurosurgical apparatus of claim 18, wherein the guide tube has an increased diameter open proximal end for seating in a corresponding shaped seat in the guide hub passage.
20. The neurosurgical apparatus of any one of claims 1 to 17, wherein the guide tube comprises an enlargement at the proximal end sized and shaped for securing in a burr hole in a skull.
21. The neurosurgical apparatus of any preceding claim, wherein the guide tube is resiliently extendible and compressible in the axial direction, at least in a proximal end portion.
22. The neurosurgical apparatus of claim 21, wherein the proximal end portion of the guide tube has a Poisson's ratio of zero or less.
23. The neurosurgical apparatus of claim 21 or claim 22, wherein the guide tube is of laminate construction, comprises an inner tube of a stiffer material overlaid with an outer layer of a porous resiliently deformable material; and wherein the proximal end portion is not provided with the inner tube.
24. The neurosurgical apparatus of any preceding claim further comprising a cannula for insertion through the guide tube into the brain, to deliver an infusate to a target brain volume.
25. The neurosurgical apparatus of claim 24, wherein the cannula comprises a bubble vent configured to prevent gas and/or micro-organisms from entering the cannula.
26. The neurosurgical apparatus of claim 25, wherein the bubble vent comprises a first membrane and a second membrane separated by an air gap, wherein the first membrane is hydrophobic and the second membrane is hydrophilic.
27. The neurosurgical apparatus of any preceding claim further comprising a probe for insertion into tissue, the probe comprising: a rod having a rounded or conical distal end provided with an axially extending, narrower diameter spike having an extreme end for dissecting tissue.
28. A cannula for insertion through a guide tube into the brain, to deliver an infusate to a target brain volume, wherein the cannula comprises a bubble vent configured to prevent gas and/or micro-organisms from entering the cannula.
29. The cannula of claim 25, 26, or 28, wherein the bubble vent is permanently joined to and/or integrally formed with the cannula.
30. A guide tube for insertion into the brain comprising: a proximal end; a distal end; and a through-bore for passage of a cannula; wherein at least an outer layer the guide tube is of a hydrophobic material that is resiliently deformable and porous to allow passage of air.
31. A package comprising the guide tube of claim 30 and a packaging tube, wherein the guide tube is provided within the packaging tube, and the packaging tube is configured to compress the outer layer of the guide tube.
32. The package of claim 31, further comprising a stylet within the through-bore of the guide tube.
33. A probe for insertion into tissue, the probe comprising: a rod having a rounded or conical distal end provided with an axially extending, narrower diameter spike having an extreme end for dissecting tissue.
34. The probe of claim 33 having a diameter of 1.3 mm or less.
35. The probe of claim 33 or claim 34 wherein the spike is from 4mm to 5mm long and tapers from 0.5mm to 0.3mm at its extreme distal end.
36. A kit for convection enhanced delivery of an infusate to the brain parenchyma comprising: a) a guide tube for insertion into the brain and having a proximal end, a distal end and a through-bore for passage of a cannula; wherein at least an outer layer of the guide tube is of a hydrophobic material that is resiliently deformable and porous to allow passage of air; and b) a guide tube probe for passing through the throughbore of the guide tube, to assist insertion of the guide tube into the brain; c) a probe for preparing a track in the brain for a cannula; and d) a cannula for passage through the guide tube to deliver an infusate to the brain.
37. The kit of claim 36 wherein the probe c) for preparing a track for a guide tube and cannula in the brain comprises: a rod having a rounded or conical distal end provided with an axially extending, narrower diameter spike having an extreme end for dissecting tissue.
38. The kit of claim 36 or claim 37 further comprising a guide hub for fitting to a burr hole in the skull and connecting to the proximal end of the guide tube.
39. A surgical method for convection enhanced delivery of an infusate to the brain parenchyma, the method comprising: a) passing a guide tube into the brain parenchyma, wherein the guide tube comprises: a proximal end; a distal end; and a through-bore for passage of a cannula; wherein at least an outer layer the guide tube is of a hydrophobic material that is resiliently deformable and porous to allow passage of air; and wherein the guide tube is passed into the brain with the aid of a guide tube probe passing through the throughbore so that its distal end is at or just beyond the distal end of the guide tube; b) when the distal end of the guide tube is at its planned position, advancing the guide tube probe further along the trajectory to create a track through the brain tissue to accommodate the cannula; c) removing the guide tube probe; d) passing a cannula through the throughbore and into the brain along the track; and e) passing an infusate into the brain via the cannula.

## Claims

1. A cannula for insertion through a guide tube into the brain, to deliver an infusate to a target brain volume, wherein the cannula comprises a bubble vent configured to prevent gas and/or micro-organisms from entering the cannula.

2. The cannula of claim 1, wherein the bubble vent is permanently joined to and/or integrally formed with the cannula.

3. The cannula of claim 1 or 2, wherein the bubble vent is provided at a proximal end of the cannula.

4. The cannula of any preceding claim, wherein the bubble vent comprises a hydrophobic, gas permeable, microporous structure.

5. The cannula of any preceding claim, wherein the bubble vent comprises at least one of:
a) a filter guard comprising a plurality of perforations configured to degas fluid flowing through the bubble vent; and
b) a proximal connector comprising a septum.

6. The cannula of any preceding claim, wherein the bubble vent comprises a first membrane and a second membrane.

7. The cannula of claim 6, wherein at least one of:
a) the first membrane is hydrophobic and gas permeable; and
b) the second membrane is hydrophilic and liquid permeable.

8. The cannula of claim 6 or 7, wherein at least one of:
a) the first membrane and the second membrane are separated by a gap of between 0.05mm and 0.2mm, preferably of 0.1mm; and
b) the first membrane and the second membrane are substantially parallel to one another.

9. The cannula of any of claims 6 to 8, wherein the bubble vent comprises a fluid passage fluidly connecting a proximal connector of the bubble vent and the cannula, and the first membrane and the second membrane are substantially perpendicular to an axis of the fluid passage.

10. The cannula of any of claims 6 to 9, wherein:
the bubble vent comprises a fluid passage fluidly connecting a proximal connector of the bubble vent and the cannula; and
the first membrane and the second membrane are positioned between a distal end of the fluid passage and a proximal end of the cannula.

11. The cannula of claim 10, wherein a hole is provided in the first membrane where the fluid passage meets the first membrane, such that fluid from the fluid passage can pass through the first membrane via the hole.

12. The cannula of claim 10 or 11, wherein the bubble vent comprises one or more vent holes on a proximal side of the first membrane, and fluid from the fluid passage must pass through the material of the first membrane to reach the vent holes.

13. The cannula of any of claims 10 to 12, wherein fluid from the fluid passage must pass through the material of the second membrane to reach the cannula.

14. A kit for convection enhanced delivery of an infusate to the brain parenchyma comprising:
a) a guide tube for insertion into the brain and having a proximal end, a distal end and a through-bore for passage of a cannula, wherein at least an outer layer of the guide tube is of a hydrophobic material that is resiliently deformable and porous to allow passage of air;
b) a guide tube probe for passing through the throughbore of the guide tube, to assist insertion of the guide tube into the brain;
c) a probe for preparing a track in the brain for a cannula; and
d) a cannula according to any one of claims 1 to 13 for passage through the guide tube to deliver an infusate to the brain.

15. The kit of claim 14, wherein at least one of:
a) the probe for preparing a track for a guide tube and cannula in the brain comprises: a rod having a rounded or conical distal end provided with an axially extending, narrower diameter spike having an extreme end for dissecting tissue; and
b) the kit further comprises a guide hub for fitting to a burr hole in the skull and connecting to the proximal end of the guide tube.
